Europäisches Patentamt

European Patent Office

Office européen des brevets

(11) Veröffentlichungsnummer: **0 255 645**
**A1**

## EUROPÄISCHE PATENTANMELDUNG

(21) Anmeldenummer: **87110501.1**

(22) Anmeldetag: **20.07.87**

(51) Int. Cl.⁴: **A61G 7/10** , A61B 6/04

(30) Priorität: **31.07.86 DE 8620535 U**

(43) Veröffentlichungstag der Anmeldung:
**10.02.88 Patentblatt 88/06**

(84) Benannte Vertragsstaaten:
**DE FR**

(71) Anmelder: **Siemens Aktiengesellschaft Berlin und München**
**Wittelsbacherplatz 2**
**D-8000 München 2(DE)**

(72) Erfinder: **Grasser, Franz, Dipl.-Ing. (FH)**
**Neuwiesenstrasse 27**
**D-8551 Eggolsheim(DE)**

(54) **Patientenlagerungstisch.**

(57) Die Erfindung betrifft einen Patientenlagerungstisch für eine Einrichtung zum Zertrümmern von Konkrementen im Körper eines Patienten (4) mit einer Lagerungsfläche (3, 3a), die eine Öffnung (5) aufweist, in der ein Stoßwellenrohr (6) zur Erzeugung von zur Zertrümmerung der Konkremente dienenden Stoßwellen angeordnet ist. Um auf einfache Weise eine exakte Positionierung des Patienten (4) relativ zu der Öffnung (5) bzw. dem Stoßwellenrohr (6) zu ermöglichen, sind Transportmittel (9, 9a) vorgesehen, mittels derer der Patient (4) auf der Lagerungsfläche (3, 3a) relativ zu der Öffnung (5) verschiebbar ist.

FIG 1

EP 0 255 645 A1

## Patientenlagerungstisch

Die Erfindung betrifft einen Patientenlagerungstisch für eine Einrichtung zum Zertrümmern von Konkrementen im Körper eines Patienten mit einer Lagerungsfläche, die eine Öffnung aufweist, in der ein Stoßwellenrohr zur Erzeugung von zur Zertrümmerung der Konkremente dienenden Stoßwellen angeordnet ist.

Ein solcher Patientenlagerungstisch ist in dem Prospekt der Fa. Siemens, "Lithostar - Universeller urologischer Arbeitsplatz für Therapie und Diagnostik", Druckzeichen:A91001-M1027-G490-01 PA 4864, beschrieben. Zur Behandlung des Patienten wird das in der Öffnung befindliche Stoßwellenrohr, das fokussierte Stoßwellen abgibt, derart akustisch an die Körperoberfläche des auf der Lagerungsfläche liegenden Patienten angekoppelt, daß sich das zu zertrümmernde Konkrement, z.B. ein Nierenstein, im Brennpunkt der Stoßwellen befindet. Dazu ist eine exakte Positionierung des Patienten relativ zu dem Stoßwellenrohr erforderlich. Bei dem bekannten Patientenlagerungstisch ist deshalb die Lagerungsfläche samt des darauf liegenden Patienten relativ zu dem Stoßwellenrohr in der Körperlängsrichtung des Patienten und quer zu dieser motorisch verschiebbar. Um ein Durchfallen des in der Regel narkotisierten Patienten durch die Öffnung zu vermeiden, weist diese jedoch in der Körperlängsrichtung des Patienten nur relativ geringe Abmessungen auf. Da außerdem während des Verschiebens der Lagerungsfläche das Stoßwellenrohr in deren Öffnung angeordnet ist, kann die Lagerungsfläche relativ zu dem Stoßwellenrohr nur um ein geringes Maß in der Körperlängsrichtung des Patienten verschoben werden, da andernfalls die Begrenzungskanten der Öffnung an dem Stoßwellenrohr anstoßen und dieses aus seiner vorbestimmten Lage verschieben oder gar beschädigen würden. Bei dem bekannten Patientenlagerungstisch müssen deshalb die behandelnden Ärzte und das Bedienungspersonal den Patienten wenigstens in seiner Körperlängsrichtung zunächst manuell schon so genau auf der Lagerungsfläche ausrichten, daß dessen exakte Positionierung innerhalb des durch die Abmessungen des Stoßwellenrohres und der Öffnung begrenzten Verstellbereiches der Lagerungsfläche motorisch erfolgen kann. Dies ist infolge des Umstandes, daß der Patient narkotisiert ist, mit erheblichen körperlichen Anstrengungen verbunden. Außerdem besteht die Gefahr, daß ein Patient, der z.B. in Querrichtung der Lagerungsfläche bereits exakt positioniert ist, wieder verschoben wird. Des weiteren ist diese Vorgehensweise für den Patienten wenig schonend, da erhebliche Kräfte auf diesen ausgeübt werden.

Der Erfindung liegt die Aufgabe zugrunde, einen Patientenlagerungstisch der eingangs genannten Art so auszubilden, daß auf einfache Weise ohne Vergrößerung der Öffnung eine exakte Positionierung des Patienten relativ zu dem Stoßwellenrohr möglich ist, ohne daß dies mit körperlicher Anstrengung für die behandelnden Ärzte und das Bedienungspersonal verbunden ist und ohne daß nennenswerte Kräfte auf den Patienten ausgeübt werden.

Nach der Erfindung wird diese Aufgabe dadurch gelöst, daß Transportmittel vorgesehen sind, mittels derer der Patient auf der Lagerungsfläche relativ zu der Öffnung verschiebbar ist. Der Patient, der wenigstens mit einem Teil seines Körpers auf den Transportmitteln aufliegt, kann somit leicht relativ zu der Öffnung verschoben und exakt positioniert werden, ohne daß dies mit besonderen körperlichen Anstrengungen für die behandelnden Ärzte und das Bedienungspersonal verbunden ist. Wenn die Transportmittel motorisch antreibbar sind, entfallen körperliche Anstrengungen vollständig. Auch für den Patienten gestaltet sich der Positionierungsvorgang schonender, da nur geringe Kräfte auf ihn ausgeübt werden. Wenn außerdem die Transportmittel einen unbegrenzten Verschiebeweg aufweisen, kann der Patient in einer nahezu beliebigen Lage auf die Lagerungsfläche aufgelegt und anschließend problemlos mittels der Transportmittel relativ zu der Öffnung positioniert werden.

Aus der DE-OS 26 19 468 ist zwar ein Computertomograph bekannt, der zwei Liegen aufweist, deren Auflagefläche jeweils mit Transportmitteln versehen ist, wobei die Liegen derart angeordnet sind, daß ihre einander zugewandten Enden in einem geringen Abstand voneinander angeordnet sind, so daß ein schmaler Spalt zum Durchtritt von Röntgenstrahlen vorhanden ist, jedoch ist der Spalt nur vorgesehen, um Abbildungen der Liegen durch die Röntgenstrahlung zu vermeiden. Irgendwelche Geräteteile, insbesondere Stoßwellenrohre, sind in dem Spalt nicht angeordnet. Sie fänden darin in Anbetracht der geringen Breite des Spaltes auch keinen Raum. Bei dem Spalt des bekannten Computertomographen handelt es sich somit nicht um eine Öffnung im Sinne der vorliegenden Erfindung, in der ein mit dem Patienten in Eingriff stehendes Teil angeordnet ist.

Eine besonders einfache Ausbildung des erfindungsgemäßen Patientenlagerungstisches wird erreicht, wenn die Lagerungsfläche durch die Transportmittel gebildet ist. Die Transportmittel können dann nach einer Variante der Erfindung durch wenigstens ein Transportband gebildet sein, wodurch auf einfache Weise eine gute Auflage des Patienten und ein unbegrenzter Verschiebeweg der Transportmittel erreicht wird. Da das Transportband Verschiebungen des Patienten nur in einer Richtung, z.B. in Körperlängsrichtung des Patienten, ermöglicht, müssen Maßnahmen getroffen werden, um den Körper des Patienten auch in der rechtwinklig dazu verlaufenden Richtung verschieben zu können, was z.B. dadurch erreicht werden kann, daß die Lagerungsfläche in dieser Richtung insgesamt motorisch verschiebbar ist. Dabei ist es zweckmäßig, die Lagerungsfläche quer zur Körperlängs richtung des Patienten motorisch verschiebbar auszubilden, da sich dann die Öffnung, ohne daß die Gefahr des Durchfallens des Patienten besteht, über die gesamte Breite der Lagerungsfläche erstrecken kann, so daß ein ausreichend großer Verschiebeweg zur Verfügung steht, um den Patienten in dieser Richtung exakt positionieren zu können.

Besonders vorteilhaft ist es, wenn die Transportmittel gemäß einer Ausführungsform der Erfindung aus zwei beiderseits der Öffnung angeordneten Transportstrecken bestehen, deren einander zugewandte Enden die Öffnung begrenzen. In diesem Falle liegt der Patient mit seinem Körper beiderseits der Öffnung auf den die jeweilige Transportstrecke bildenden Transportmitteln auf. Da diese beim Positionieren des Patienten gleichsinnige Bewegungen ausführen, können keine Reibungskräfte zwischen dem Körper des Patienten und der Lagerungsfläche entstehen, die einer leichten Verschiebbarkeit und einer exakten Positionierbarkeit des Patienten entgegenwirken könnten. Sofern die Transportmittel motorisch antreibbar sind, kann es bereits genügen, wenn wenigstens eine Transportstrecke motorisch antreibbar ist. Falls vorgesehen ist, daß beide Transportstrecken motorisch antreibbar sind, ist es zweckmäßig, diese zum Positionieren des Patienten synchron und gleichsinnig motorisch anzutreiben, da dann unnötige Beanspruchungen des Körpers, insbesondere aber der Haut des Patienten vermieden werden. Eine Ausführung der Erfindung sieht vor, daß außerdem beide Transportstrecken unabhängig voneinander motorisch antreibbar sind. Indem nur eine der beiden Transportstrecken motorisch angetrieben und die andere gebremst wird oder beide Transportstrecken gegensinnig angetrieben werden, ist es nämlich möglich, die im Bereich der Öffnung befindliche Haut des Patienten zu straffen, was für eine besonders wirksame akustische Ankopplung des Stoßwellenrohres an den Körper des Patienten förderlich ist.

In der beigefügten Zeichnung ist ein Ausführungsbeispiel der Erfindung dargestellt. Es zeigen:

Fig. 1 einen Längsschnitt nach der Linie I-I in Figur 2 durch einen erfindungsgemäßen Patientenlagerungstisch, und

Fig. 2 eine Ansicht des erfindungsgemäßen Patientenlagerungstisches.

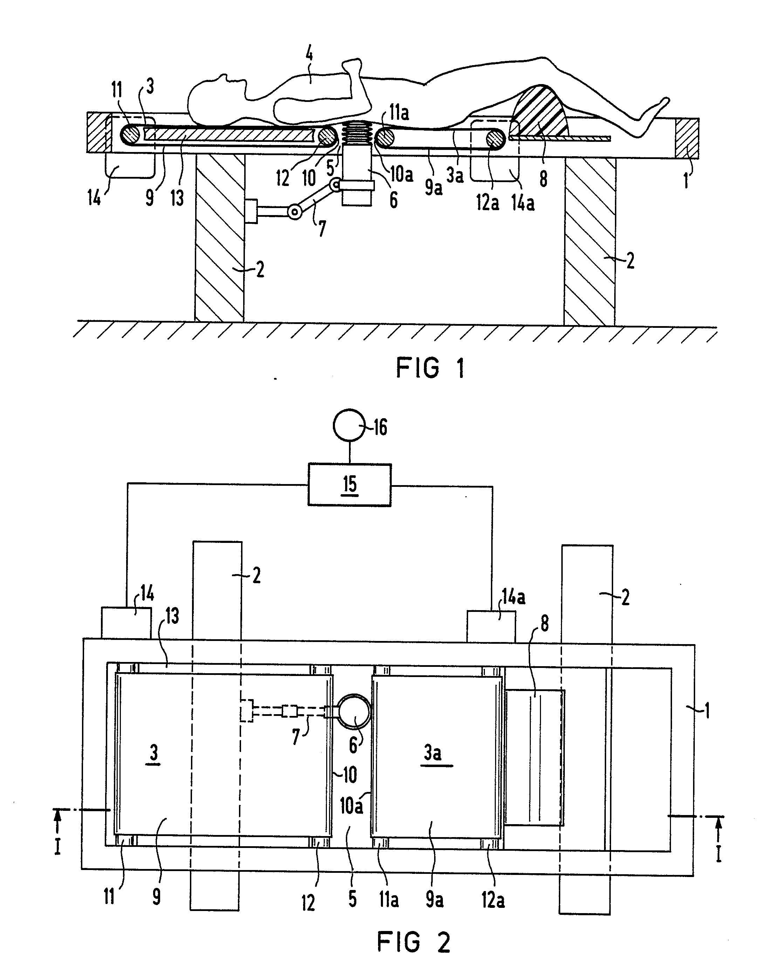

Die Figuren 1 und 2 zeigen einen erfindungsgemäßen Patientenlagerungstisch, der Teil einer Einrichtung zum Zertrümmern von Konkrementen im Körper eines Patienten 4 ist, der einen Rahmen 1 mit Ständern 2 und mit einer Lagerungsfläche 3, 3a für einen aus Gründen der Übersichtlichkeit in Figur 2 nicht dargestellten Patienten 4 aufweist. Die Lagerungsfläche 3, 3a weist eine Öffnung 5 auf, durch die ein Bereich der Körperoberfläche des Patienten 4 zur Behandlung mit einem Stoßwellenrohr 6 zugänglich ist. Dieses ist mittels eines Haltearmes 7 an einem der Ständer 2 befestigt. Das Stoßwellenrohr 6 ist akustisch mit dem Körper des Patienten 4 gekoppelt und gibt fokussierte Stoßwellen ab, die zur Zerstörung von im Körper des Patienten 4 befindlichen Konkrementen dienen. Zu diesem Zweck muß der Patient 4, dessen Beine durch ein Polster 8 unterstützt sind, auf der Lagerungsfläche 3, 3a relativ zu der Öffnung 5 und dem darin befindlichen Stoßwellenrohr 6 so positioniert sein, daß sich die zu zertrümmernden Konkremente im Brennpunkt der Stoßwellen befinden.

Der erfindungsgemäße Patientenlagerungstisch weist deshalb zur Verschiebung des Patienten 4 relativ zu der Öffnung 5 Transportmittel auf, die aus zwei beiderseits der Öffnung 5 angeordneten Transportstrecken bestehen, deren einander zugewandten Enden 10, 10a die Öffnung 5 begrenzen. Die Transportstrecken sind als Transportbänder 9, 9a ausgebildet, die über drehbar in dem Rahmen 1 gelagerte Rollen 11, 12 und 11a, 12a in Körperlängsrichtung des Patienten 4 laufen und somit eine Positionierung des Patienten 4 relativ zu der Öffnung 5 in dieser Richtung gestatten. Die die Öffnung 5 begrenzenden Enden 10 und 10a der Transportstrecken sind durch die Rollen 12 und 11a und die über diese laufenden Transportbänder 9 und 9a gebildet. Verschiebungen des Patienten 4 relativ zum Stoßwellenrohr 6 quer zur Körperlängsrichtung des Patienten 4 sind dadurch möglich, daß der Rahmen 1 samt der Lagerungsfläche 3, 3a in nicht dargestellter Weise motorisch verschiebbar auf den Ständern 2 angebracht ist.

Die Lagerungsfläche 3, 3a ist unmittelbar durch die Transportbänder 9, 9a gebildet. Dabei wird der Patient 4 im Bereich seines Oberkörpers außer durch das Transportband 9 durch eine zwischen dessen Trumen vorgesehene Platte 13 unterstützt, während er im Bereich seines Unterkörpers ausschließlich durch das Transportband 9a getragen wird.

Die Transportbänder 9 und 9a sind durch Motore 14, 14a, die mit den Rollen 11 und 12a gekuppelt sind, motorisch antreibbar. Zu diesem Zweck sind sie, wie aus Figur 2 ersichtlich ist, über eine Steuereinrichtung 15 mit einer Stromquelle 16 verbunden. Die Steuereinrichtung 15 ist derart ausgebildet, daß wahlweise allein das Transportband 9 oder 9a mittels des jeweils zugehörigen Motors 14 oder 14a antreibbar ist. Außerdem gestattet es die Steuereinrichtung 15 beide Transportbänder 9, 9a mittels der Motore 14, 14a synchron und gleichsinnig anzutreiben.

Im Falle des beschriebenen Ausführungsbeispieles sind die Transportmittel als Transportbänder ausgebildet. Es ist jedoch auch möglich, die Transportmittel durch eine Vielzahl von Rollen, Kugeln oder dergleichen zu bilden.

Außerdem kann bei geeigneter Anordnung und Ausbildung der Transportmittel der Körper des Patienten im Gegensatz zu dem dargestellten Ausführungsbeispiel auch quer zu seiner Körperlängsachse oder in beliebigen Richtungen relativ zu der Öffnung verschoben werden.

Im Falle des beschriebenen Ausführungsbeispieles wird der erfindungsgemäße Patientenlagerungstisch beim Zertrümmern von im Körper eines Patienten befindlichen Konkrementen benutzt. Er ist jedoch für alle Diagnose-und Therapieverfahren verwendbar, bei denen ein Bereich der Körperoberfläche des Patienten durch eine Öffnung der Lagerungsfläche zugänglich sein muß und eine Verschiebbarkeit des Patienten relativ zu der Öffnung erforderlich ist.

**Ansprüche**

1. Patientenlagerungstisch für eine Einrichtung zum Zertrümmern von Konkrementen im Körper eines Patienten (4) mit einer Lagerungsfläche (3, 3a), die eine Öffnung (5) aufweist, in der ein Stoßwellenrohr (6) zur Erzeugung von zur Zertrümmerung der Konkremente dienenden Stoßwellen angeordnet ist, **dadurch gekennzeichnet**, daß Transportmittel (9, 9a) vorgesehen sind, mittels derer der Patient (4) auf der Lagerungsfläche (3, 3a) relativ zu der Öffnung (5) verschiebbar ist.

2. Patientenlagerungstisch nach Anspruch 1, **dadurch gekennzeichnet**, daß die Transportmittel durch wenigstens ein Transportband (9, 9a) gebildet sind.

3. Patientenlagerungstisch nach Anspruch 1 oder 2, **dadurch gekennzeichnet**, daß die Transportmittel aus zwei beiderseits der Öffnung (5) angeordneten Transportstrecken (9 und 9a) bestehen, deren einander zugewandte Enden (10 und 10a) die Öffnung (5) begrenzen.

4. Patientenlagerungstisch nach Anspruch 3, **dadurch gekennzeichnet**, daß beide Transportstrecken (9, 9a) synchron und gleichsinnig motorisch antreibbar sind.

5. Patientenlagerungstisch nach Anspruch 4, **dadurch gekennzeichnet**, daß beide Transportstrecken (9, 9a) außerdem unabhängig voneinander motorisch antreibbar sind.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | DE-A-2 619 468 (SIEMENS AG) * Seite 4, Zeilen 6-9,17-22; Seite 5, Zeilen 1-18; Seite 6, Zeilen 1-3; Figuren 1,2 * | 1-4 | A 61 G 7/10 A 61 B 6/04 |
| | --- | | |
| X | DE-A-2 619 482 (SIEMENS AG) * Seite 5, Zeilen 9-11,19-25; Seite 6, Zeilen 3-19; Seite 7, Zeilen 1-4; Figuren 1,2 * | 1-4 | |
| | --- | | |
| A | DE-U-8 322 413 (SIEMENS AG) * Zusammenfassung * | 1 | |
| | ----- | | |

| RECHERCHIERTE SACHGEBIETE (Int. Cl.4) |
|---|
| A 61 G A 61 B |

Der vorliegende Recherchenbericht wurde für alle Patentansprüche erstellt.

| Recherchenort | Abschlußdatum der Recherche | Prüfer |
|---|---|---|
| DEN HAAG | 06-11-1987 | BAERT F.G. |